Europäisches Patentamt

European Patent Office

Office européen des brevets

(19)

(11) Veröffentlichungsnummer: **0 000 759**
**B1**

(12) **EUROPÄISCHE PATENTSCHRIFT**

(45) Veröffentlichungstag der Patentschrift: 02.09.81

(51) Int. Cl.³: **A 61 N 1/04, A 61 B 5/04**

(21) Anmeldenummer: **78100552.5**

(22) Anmeldetag: **31.07.78**

(54) Elektrode.

(30) Priorität: **03.08.77 DE 2735050**
**03.08.77 DE 2735041**
**14.07.78 DE 2831109**
**14.07.78 DE 2831099**

(43) Veröffentlichungstag der Anmeldung:
**21.02.79 Patentblatt 79/4**

(45) Bekanntmachung des Hinweises auf die Patenterteilung:
**02.09.81 Patentblatt 81/35**

(84) Benannte Vertragsstaaten:
**BE DE FR GB NL SE**

(56) Entgegenhaltungen:
**DE - A - 2 414 584**
**DE - A - 2 459 627**
**DE - A - 2 629 549**
**DE - A - 2 754 465**
**FR - A - 1 098 726**
**US - A - 3 566 860**
**US - A - 3 606 881**
**US - A - 3 696 807**
**US - A - 3 828 766**
**US - A - 3 888 240**

(73) Patentinhaber: **SIEMENS AKTIENGESELLSCHAFT**
**Berlin und München**
**Postfach 22 02 61**
**D-8000 München 22 (DE)**

(72) Erfinder: **Szehi, Erich**
**Am Eichengarten 11**
**D-8520 Erlangen (DE)**
Erfinder: **Naser, Georg**
**Karlstrasse 10**
**D-8502 Zirndorf (DE)**

Courier Press, Leamington Spa, England.

Elektrode

Die Erfindung bezieht sich auf eine Elektrode zur Abnahme oder Zuführung von elektrischen Signalen von oder zum Körper eines Patienten mit wenigstens einem Kontaktteil aus einem elektrisch leitenden Kunststoff-Schaumstoffeinsatz. Eine derartige Elektrode ist aus der FR—A—1 098 726 bekannt.

Elektroden dieser Art lassen sich zur Abnahme physiologischer Signale, wie EKG od.dgl., einsetzen. Ebensogut können über solche Elektroden einem Körper jedoch auch elektrische Signale, beispielsweise Reizstrom bei der Reizstrombehandlung, zugeführt werden. Wesentlich ist aber immer, daß bei Applikation die Elektroden am Körper des Patienten gut kontaktieren, so daß Signale vom Patientenkörper möglichst störungsfrei abgenommen werden können oder dem Patientenkörper über die Elektroden störungsfrei Strom zugeführt werden kann.

Insbesondere bei großflächigen Elektroden, die speziell auch an Körperpartien eines Patienten mit unterschiedlichen bzw. wechselnden Wölbungen appliziert werden sollen, besteht das Bedürfnis, das Kontaktteil zur Anlage an die Körperoberfläche zwecks Anpassung an die Körperkontur flexibel und möglichst weich auszubilden. Das gleiche gilt aber auch für die Kontaktteile von Saugelektroden, wie sie beispielsweise aus der DE—B—12 24 847 vorbekannt sind. Speziell bei Saugelektroden haftet das Saugnapfgehäuse samt Elektrode an der Applikationsstelle, wobei zur Herstellung einer leitenden Verbindung zwischen Körperoberfläche und eigentlichen Elektrodenkontaktteil eine elastische Platte aus Natur- oder Viskoseschwamm mit Kontaktflüssigkeit dient.

Vom Stand der Technik (beispielsweise DE—A—26 29 549, US—A—38 28 766, US—A—36 96 807, DE—A—24 59 627) sind darüber auch bereits Elektroden bekanntgeworden, bei denen speziell Schaumstoffe insätze Bestandteile der Elektrodenkontaktteile sind. Diese vorbekannten Schaumstoffeinsätze dienen aber regelmäßig als Träger für ein zusätzliches Kontaktmittel, welches sie nach Art eines Schwammes speziell als flüssigen Elektrolyten oder als Elektrodengel aufnehmen. Insofern entsprechen diese Elektrodenkontaktteile wiederum den mit Flüssigkeit getränkten Schwammplatten bei der vorbekannten Saugelektrode.

Beim obigen Stand der Technik sind zwar bereits die Forderungen hinsichtlich der Weichheit des Kontaktteiles erfüllt, wobei im allgemeinen dei Elektroden zusätzlich applikationsseitig abgepolstert sind; andererseits wird aber immer das zusätzliche Kontaktmittel zur Kontaktierung benötigt. Speziell bei der erwähnten Saugelektrode ergibt sich dadurch aber weiterhin der Nachteil, daß aufgrund des Saugprinzips permanent Kontaktflüssigkeit durch den Zuführungsschlauch von der Saugpumpe angesaugt wird, wodurch sich die Kontaktflüssigkeit im Zuführungsschlauch und in der auf Erdpotential liegenden Schlauchpumpe sammeln kann; abgesehen von der unerwünschten Verschmutzung können sich also dadurch unerwünschte elektrische Nebenschlüsse bilden.

Speziell vorstehend genannter Nachteile ist zwar bereits bei solchen Saugelektroden beseitigt, die nach dem Luftstrahlpumpenprinzip (Injektorprinzip) arbeiten und beispielsweise aus der DE—B—19 39 523 vorbekannt sind. Bei diesen Elektroden wird die angesaugte Kontaktflüssigkeit von der Strömung des den Unterdruck erzeugenden genden Preßgases von der Strahlpumpe weg ins Freie gesprüht. Die Kontaktflüssigkeit kann also nicht mehr in die Leitungen zur Saugpumpe gelangen, so daß elektrische Nebenschlüsse dort auch nicht mehr auftreten können. Trotz der erheblichen Vorteile der Saugelektrode nach dem Luftstrahlpumpenprinzip ergeben sich aber noch gewisse Nachteile. Bedingt durch die kleinen Düsenquerschnitte zwischen Unterdruckraum und Strahlrohr im Saugnapfgehäuse kommt es schon bei geringen Verunreinigungen relativ rasch zu Verengungen. Hier besteht also die Gefahr, daß die Saugelektroden sich lockern und abfallen.

Als Schaumstoffeinsätze werden beispielsweise speziell bei der DE—A—26 19 549 sowie auch bei der US—A—38 28 766 offenzellige Polyurethan-Schaumstoffe verwendet. Wesentlich ist dabei aber immer die Offenporigkeit des Schaumstoffes zur Aufnahme des flüssigen Elektrolyten zwecks Bildung eines Schwammeinsatzes für das Kontaktmittel. Über den oben abgehandelten Stand der Technik hinaus ist weiterhin aus der FR—A—10 98 726 eine Elektrode bakannt, bei der das Kontaktteil durch ein Formteil aus leitfähig gemachten Silikonkautschuk gebildet ist, das zusätzlich an seiner Oberfläche aufgeschäumt wird. Die dadurch gebildeten Poren dienen aber wieder zur Aufnahme einer Flüssigkeit als zusätzliches Kontaktmittel.

Der Erfindung liegt daher die Aufgabe zugrunde, eine Elektrode zu schaffen, die den Einsatz von zusätzlichen Kontaktmitteln überflüssig macht. Solche Elektroden sollen alternativ als großflächige Einzelelektrode, als Mehrfachelektrode oder auch als Saugelektrode realisierbar sein. Insbesondere für letzteren Fall soll auch erreicht werden, daß sich derartige Elektroden nicht aufgrund Verunreinigungen der Düsen durch Kontaktmittel, die dadurch das Saugvermögen herabsetzen, unbeabsichtigt lockern oder abfallen können.

Die Aufgabe wird erfindungsgemäß dadurch gelöst, daß der Kunststoff-Schaumstoffeinsatz auf der Applikationsseite eine glatte, porenverschlossene Oberfläche als Kontaktfläche aufweist, die zur Herstellung eines elektrischen Kontaktes mit dem Körper des Patienten ohne zusätzliche Anwendung von separaten Kontaktmitteln geeignet ist.

In erster Ausführungsform der Erfindung kann der Kunststoff-Schaumstoffeinsatz aus einem geschlossenporigen Schaumstoff aus leitfähigem Basismaterial bestehen. In zweiter Ausführungsform kann aber der Kunststoff-Schaumstoffeinsatz aus einem offenporigen, nach dem Aufschäumen beleitfähigtem Schaumstoff bestehen, dessen als Kontaktfläche vorgesehene Oberfläche eine porenverschließende Lackierung (sog. Coating) aufweist.

Die erfindungsgemäß ausgebildeten Kunststoff-Schaumstoffe haben einen spezifischen elektrischen Widerstand kleiner als 2000 Ohm·cm und eine Stauchhärte zwischen 1 und 20 kPa. Kunststoffe für die erste Ausführungsform können beispielsweise leitfähiggemachter Silikonkautschuk, für die zweite Ausführungsform beispielsweise Polyurethan, wie Polyäther oder Polyester, Polyäthylene, Polyvinylchloride oder Polyamide sein. In einem Fall wird also das leitfähige Basismaterial aufgeschäumt, während im letzteren Fall die Basismaterialien, die offenporig geschäumt werden, in einfacher Weise beleitfähigt werden.

Bei der Erfindung wird also ein trocken leitfähiger Kunststoff-Schaumstoffeinsatz als Elektrodenkontaktteil verwendet. Im Gegensatz zu den üblichen Kontaktierungsmitteln, wie z.B. mit Kontaktflüssigkeit getränkte Schwämme oder Schaumstoffe, weist der erfindungsgemäße Kunststoff-Schaumstoffeinsatz bereits ohne zusätzliche Kontaktflüssigkeit ausgezeichnete elektrische Leitfähigkeit auf. Auf die Tränkung mit einer solchen Kontaktflüssigkeit kann also von vornherein verzichtet werden, wodurch sich die Applikation bei ausgezeichneter Kontaktierung erheblich erleichtert. Der erfindungsgemäße Kunststoff-Schaumstoffeinsatz besitzt aber neben guter Leitfähigkeit auch ausgezeichnete Elastizität, so daß ein gutes Anliegen der gesamten Elektrodenfläche auf der Haut gewährleistet ist. Hieraus wiederum resultiert erhöhte Störsicherheit bei der Abnahme bzw. Zuführung der elektrischen Signale.

Erfindungsgemäße Elektroden lassen sich als Einzelelektrode oder auch als Mehrfachelektrode einsetzen. In der Ausbildung als Mehrfachelektrode ist dabei eine der gewünschten Zahl der Kontaktstellen entsprechende Anzahl von Kunststoff-Schaumstoffeinsätzen auf einem gemeinsamen Elektrodenträger angeordnet. In vorteilhafter Ausgestaltung sind dann die Kunststoff-Schaumstoffeinsätze applikationsseitig mit einem gemeinsamen Elektrodenpapier abgedeckt. Die von Kunststoff-Schaumstoffeinsätzen freien Zwischenräume zwischen Elektrodenträger und gemeinsamen Elektrodenpapier sind dann vorzugsweise durch nichtleitende Einsätze, z.B. aus Moosgummi, ausgefüllt.

In spezieller Ausbildung der Erfindung als Saugelektrode ist besonders vorteilhaft, daß die Verunreinigung der Saugdüsen speziell aufgrund von zusätzlicher Kontaktflüssigkeit von vornherein nicht mehr gegeben ist. Darüber hinaus ist aber auch die Möglichkeit einer Verunreinigung durch sonstige Flüssigkeiten, z.B. auch Schweiß des transpirierenden Patienten, stark herabgesetzt bzw. ganz unterbunden, da Kunststoff-Schaumstoff mit porenverschlossener Oberfläche nicht saugfähig ist. Die besonderen Ausführungsarten der Erfindung sind im einzeluen aus den Unteransprüchen ersichtlich.

Weitere Vorteile der Erfindung ergeben sich aus der nachfolgenden Beschreibung von Ausführungsbeispielen anhand der Zeichnung.

Es zeigen:

Fig. 1 eine Einfachelektrode gemäß der Erfindung, teilweise im Schnitt,

Fig. 2 eine Mehrfachelektrode in Draufsicht und Seitenansicht,

Fig. 3 eine Detailvergrößerung des Bereichs A in Fig. 2,

Fig. 4 eine als Saugelektrode ausgebildete erfindungsgemäße Elektrode in Seitenansicht, teilweise im Schnitt.

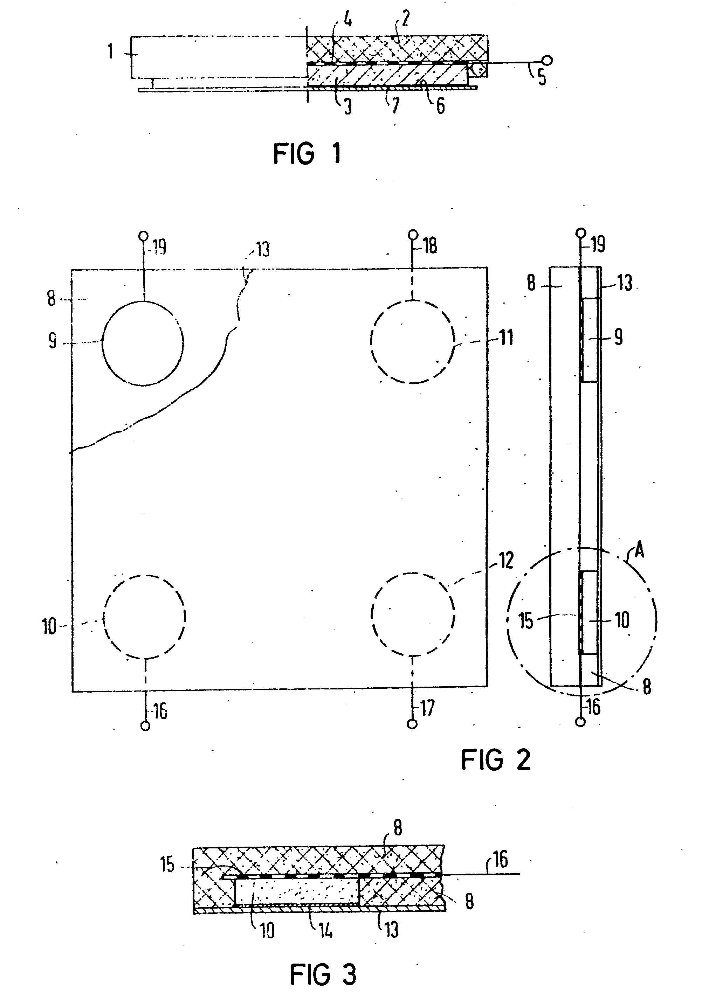

In der Fig. 1 ist in einem Elektrodengehäuse 1 ein Elektrodenträger 2 aus nichtleitendem Moosgummi angeordnet, der zur Gehäuseöffnung hin einen Kunststoff-Schaumstoffeinsatz 3 trägt. Zwischen Moosgummi 2 und Schaumstoffeinsatz 3 liegt eine Metallgaze 4 als großflächiger Anschlußkontakt für eine elektrische Leitung 5, über die ein Strom abgenommen oder zugeführt werden kann. Der Kunststoff-Schaumstoffeinsatz 3 ist bei offenporiger Schäumung desa Basismaterials an seiner Applikationsfläche 6 durch oberflächlich porenverschließende Lackierung (sogenanntes coating) glatt ausgebildet. Die glatte Fläche 6 dient zur Aufnahme eines Elektrodenpapiers 7, das im angefeuchteten Zustand durch Adhäsion gut haften bleibt. Das Elektrodenpapier, das vorzugsweise aus saugfähigem, 0,4 mm dickem Zellstoff (Vlies) besteht, nimmt nur die zur Herabsetzung de Elektrodenwiderstandes notwendige Flüssigkeitsmenge auf.

Die Mehrfachelektrode der Fig. 2 umfaßt insgesamt vier Kunststoff-Schaumstoffeinsätze 9 bis 12, die im Abstand voneinander an einem gemeinsamen Elektrodenträger 8, bei dem es sich vorzugsweise wiederum um nichtleitenden Moosgummi handelt, gehaltert sind. Sämtliche Schaumstoffeinsätze 9 bis 12 sind entsprechend dem Einsatz der Elektrode der Fig. 1 ausgebildet, d.h. sie besitzen glatte Applikationsflächen aufgrund oberflächlich porenverschließender Lackierung. Sämtliche Schaumstoffeinsätze sind ferner an der Applikationsseite mit einem gemeinsamen Elektrodenpapier 13, z.B. wiederum Vlies, abgedeckt. Jeder Schaumstoffeinsatz 9 bis 12 ist ferner mit einem Metallgazegeflecht großflächig kontaktiert. Jedes Metallgeflecht ist mit einer eigenen Leitung zur Stromabnahme bzw. Stromzuführung versehen. In der Fig. 2 und auch in der Detailvergrößerung der Fig. 3 ist eine solche Metallgaze speziell für den Schaumstoffeinsatz 10 mit 15 bezeichnet. Die zugehörige Stromleitung ist mit 16 gekennzeichnet. Die Stromleitungen der restlichen drei Kunststoff-Schaumstoffeinsätze sind in der Fig. 2 mit 17, 18 und 19 angedeutet. In der Detailvergrößerung der Fig. 3 ist außerdem die papiertragende lackierte Applikationsfläche des Kunststoff-Schaumstoffeinsatzes 10 mit 14 bezeichnet. Aus de Detailvergrößerung geht auch hervor, daß die von Kunststoff-Schaumstoffeinsätzen 9 bis 12 freien Zwischenräume zwischen Elektrodenträger 8 und gemeinsamem Elektrodenpapier 13 durch nichtleitende Einsätze, vorzugsweise ebenfalls aus Moosgummi, ausgefüllt sind, die eine gut federnde Kontaktierung ohne Bruchgefahr des Elektrodenpapiers 13 gewährleisten.

Die Einfachelektrode der Fig. 1 eignet sich insbesondere zum Einsatz bei der Abnahme eines EKG oder sonstiger physiologischer Körpersignale. Die Mehrfachelektrode der Fig. 2 wird hingegen bevorzugt bei der Reizstrombehandlung (Diagnostik und Therapie) eingesetzt, wo beispielsweise zur Erzeugung eines Interferenzstromfeldesdem Patientenkörper gleichzeitig mehrere zu überlagernde Reizströme zugeführt werden sollen. Ausgleichsströme zwischen den Kunststoff-Einsätzen sind bei entsprechend dünnem Elektrodenpapier vernachlässigbar.

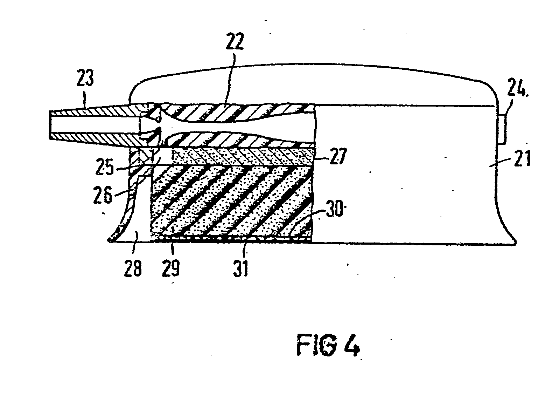

Die Saugelektrode nach Fig. 4 arbeitet speziell nach dem Luftstrahlpumpenprinzip. Die Saugelektrode umfaßt einen Saugnapf 21 als Elektrodengehäuse, der beispielsweise eine im wesentlichen zylindrische Form aufweist, die durch leichtes Zusammendrücken des elastischen Mantels im Applikationsbereich gut an die Krümmung der Körperoberfläche angepaßt werden kann. Im oberen Teil des Saugnapfgehäuses 21 befindet sich das Strahlrohr 22 der Luftstrahlpumpe mit einem Anschlußstück 23 (vorzugsweise Steckkonus) für den Schlauch zu einem (nicht dargestellten) Preßgaserzeuger und mit einem freien Auslauf 24 für das Preßgas der Strahlpumpe. Das Strahlrohr 22 der Luftstrahlpumpe ist vorzugsweise ein Kunststoffspritzteil; im Gegensatz zu Rohren aus metall wird hierdurch jede Art von Korrosion (Zersetzungserscheinungen der metallischen Strahlpumpe aufgrund elektrolysierender Vorgänge) vermieden und somit auch von dieser Seite her die Beibehaltung kleiner Düsenquerschnitte garantiert. Durch ideale Formgestaltung, die sich bei Kunststoffbearbeitung leichter als bei Metallbearbeitung erreichen läßt, kann außerdem der Wirkungsgrad der Luftstrahlpumpe aus Kunststoff gegenüber jenen aus Metall gesteigert werden. Im Ausführungsbeispiel nach Fig. 4 gemäß der Zeichnung ist der Innenraum des Strahlrohres 22 über eine enge Düse 25 sowie eine Bohrung 26 in einer Elektrodenträgerplatte 27 mit dem Unterdruckraum 28 des Saugnapfgehäuses 21 verbunden. Die Trägerplatte 27 besteht aus leitendem Material, vorzugsweise aus Graphit oder Leitgummi; sie kann jedoch ebensogut auch aus Metall gefertigt sein. Die leitende Elektrodenträgerplatte 28 trägt nun anstelle des bisher üblichen auswechselbaren Filz- oder Viskoseschwammes einen nicht oder nur wenig saugfähigen, aber gut elektrisch leitenden Kunststoff-Schaumstoffeinsatz 29. Der Schaumstoffeinsatz 29 ragt im Richtung der Applikationsöffnung des Saugnapfgehäuses 21 und er ist bei offenporiger Schäumung des Basismaterials an seiner Applikationsfläche 30 durch oberflächlich porenverschließende Lackierung glatt ausgebildet. Die glatte Fläche 30 dient zur Aufnahme eines Elektrodenpapiers 31, das in angefeuchtetem Zustand durch Adhäsion gut haften bleibt. Das Elektrodenpapier, das vorzugsweise aus saugfähigem, 0,4 mm dickem Zellstoff (Vlies) besteht, nimmt nur die zur Herabsetzung des Elektrodenwiderstandes notwendige Flüssigkeitsmenge auf. Da es sich bei der angelegten Elektrode um ein praktisch abgeschlossenes System handelt, kann die Transpiration der Haut zusätzlich zur Kontaktierung beitragen. Ein Absaugen großer Mengen überschüssiger Kontaktflüssigkeit oder auch von Körperschweiß sowie ein damit verbundenes Mitführen von Verschmutzungen wird jedoch auf jeden Fall von vornherein vermieden. Die hygienische Applikation wird ferner erheblich verbessert, wenn als Elektrodenpapier billiges Einmalpapier verwendet wird. So können Hautschuppen oder sonstige Ablagerungen nach jeder Behandlung mit dem Wegwerfen des Elektrodenpapiers beseitigt werden. Auch so wird eine mögliche Quelle für ein Verstopfen den Ansaugdüse 25 beseitigt.

Die Saugelektrode der Fig. 4 eignet sich beispielsweise für die Reizstrombehandlung (Diagnostik und Therapie); sie läßt sich ebensogut auch zur Abnahme eines EKG oder sonstiger physiologischer Körpersignale einsetzen. Die Zuführung bzw. Abnahme der elektrischen Ströme zu bzw. von der aus Kunststoff-Schaumstoffeinsatz 29, Trägerteil 27 und Elektrodenpapier 31 bestehenden Elektrode erfolgt über das Schlauchanschlußstück 23, das für den Stromübertritt zur Trägerplatte 27 metallisch ausgebildet ist.

Das Ausführungsbeispiel nach Fig. 4 beinhaltet speziell eine Saugelektrode nach dem Luftstrahlpumpenprinzip. Eine derartige Saugelektrode gewährleistet besonders gute Haftung, sofern die engen Saugdüsen im erfindungsgemäßen Sinne immer offen bleiben. Selbstverständlich läßt sich jedoch die flüssigkeitsreduzierende Applikation mit leitendem Schaumstoff auch bei Saugnapfelektroden mit geräteseitig untergebrachter Saugpumpe einsetzen. Da praktisch keine Kontaktflüssigkeit angesaugt wird, ist die Gefahr einer Ansammlung überschüssiger Kontaktflüssigkeit in den Saugleitungen bzw. in der Saugpumpe beseitigt und die damit verbundenen Nachteile können nicht mehr auftreten.

Als Materialien für die Schaumstoffeinsätze 3, 9 bis 12 und 29 werden schaumfähige, weichelastische Kunststoffe verwendet.

Unter Schaumstoffen versteht man nach DIN 7626/1 einen künstlich hergestellten, spezifisch leichten Werkstoff mit zelliger Struktur. Die Eigenschaften speziell der Schaumkunststoffe werden sowohl durch die Art der Grundmaterialien wie durch die Porenstruktur bestimmt. Bei geschlossenzelligen Schaumstoffen sind die einzelnen Luft- oder Gasbläschen gegeneinander abgeschlossen, während sie bei offenzelligen Schaumstoffen untereinander in Verbindung stehen. Dazwischen liegen mit kontinuierlichem Übergang von der einen Gruppe zur anderen Gruppe die gemischtzelligen Schaumstoffe. In der Praxis spricht man eher von vorwiegend offenporigen oder vorwiegend geschlossenporigen Schaumstoffen. Das Porenvolumen, d.h. der prozentuale Volumenanteil der Bläschen (Vakuolen) vom Gesamtvolumen, beträgt im allgemeinen immer über 50% und geht bis 99%; es ist eine wesentliche signifikante Kenngröße für die mechanischen Eigenschaften des Schaumstoffes. Je nach Herstellungsart, Größe des Volumenanteils und Basismaterials verfügt man dementsprechend über eine Reihe verschiedenartiger Schaumstoffe, die von sprödhart über zähhart bis weichelastisch führt. Für die erfindungsgemäße Verwendung als leitender Einsatz für Elektroden werden weichelastische Schaumstoffe benötigt; als Maß für die Weichheit, d.h. die flexiblen Eigenschaften der Schaumstoffe, wird zweckmäßigerweise die sog. Stauchhärte nach DIN 53577 ermittelt. Die Stauchhärte ist als die zu einer festgelegten Verformung (im allgemeinen 40%) beim Belastungsvorgang ermittelte Druckspannung definiert; sie wird in Kilo-Pascal (kPa) oder Newton pro mm² (1 kPa = 0,001 N/mm²) gemessen.

Bei der Herstellung von leitfähigen Schaumstoffen lassen sich zwei Gruppen unterscheiden:

Die erste Gruppe sind die überwiegend offenporigen Schaumstoffe. Diese werden beispielsweise aus Polyurethanen, wie Polyester und Polyäther, Polyäthylenen, Polyvinylchloriden oder Polyamiden als Basismaterial aufgeschäumt und anschließend in den offenen Poren beleitfähigt. Dafür wird ein elektrisch leitender Lack, vorzugsweise auf Kohlenstoffbasis (sog. Coatings), in die Schaumstoffe eingebracht, so daß leitfähige Teilchen an den Zellwänden haften bleiben. Insgesamt ergibt sich dadurch eine integrale Leitfähigkeit des Schaumstoffes; der spezielle Wert der elektrischen Leitfähigkeit bzw. spezifische Widerstand ergibt sich dabei aus dem Verhältnis der mit Lack beschichteten Grenzflächen der Poren zum Gesamtvolumen des Schaumstoffes. Als Parameter geht also wesentlich das bei der Herstellung des Schaumstoffes gezielt beeinflußbare Porenvolumen ein. Anderseits bestimmen — wie oben erwähnt — genau diese Parameter auch die Weichheit bzw. Flexibilität des Schaumstoffes. Die so auf Polyurethan-, Polyäthylen-, Polyvinylchlorid- und Polyamid-Basis hergestellten Schaumstoffe weisen einen spezifischen elektrischen Widerstand im Bereicha kleiner als 2000 Ohm · cm und eine Stauchhärte von 1 bis 20 kPa auf. Dabei wird der spezifische Widerstand in Anlehnung an DIN 53482 und die Stauchhärte nach DIN 53577 gemessen.

Die zweite Gruppe von Schaumstoffen kann schon durch dispergierte leitfähige Teilchen bereits als Ausgangslösung elektrisch leitend gemacht werden. Als Basismaterialien hierfür können sowohl die obengenannten Stoffe, die überwiegend offenporige Schaumstoffe bilden als auch solche Stoffe, die überwiegend geschlossenporige Schaumstoffe bilden, verwendet werden.

Beispielsweise werden bei Silikonkautschuk als Basismaterial Graphitteilchen dispergiert. Es sind leitfähige Silikonkautschuke mit spezifischen Widerständen kleiner als 20 Ohm · cm bekannt. Solche Kunst-Kautschuke können geschäumt werden. Ein geschlossenporiger Schaumstoff auf Silikonbasis hat wegen der glatten Oberflächen für die erfindungsgemäße Anwendung bei Elektroden sogar Vorteile; er braucht nicht in einem separaten Verfahrensschritt an der Oberfläche unter Porenverschluß glatt gemacht zu werden. Die Weichheit bzw. die elastischen Eigenschaften eines so hergestellten Schaumstoffs hängen wiederum im wesentlichen vom Porenvolumen ab. Im gewissen Maße werden auch noch die Menge und Größe der im Basismaterial dispergierten elektrisch leitenden Teilchen die flexiblen Eigenschaften beeinflussen. Insgesamt haben zwar die so hergestellten Schaumstoffe einen höheren spezifischen elektrischen Widerstand als das leitfähige Basismaterial; sie liegen aber bezüglich derr elektrischen Eigenschaften günstiger als die beleitfähigen Schaumstoffe.

**Patentansprüche**

1. Elektrode zur Abnahme oder Zuführung von elektrischen Signalen vom oder zum Körper eines Patienten mit wenigstens einem Kontaktteil aus einem elektrisch leitenden Kunststoff-Schaumstoffeinsatz (3; 9—12; 29), dadurch gekennzeichnet, daß der Kunststoff-Schaumstoffeinsatz (3; 9—12; 29) auf der Applikationsseite eine glatte, porenverschlossene Oberfläche als Kontaktfläche (6; 14; 30) aufweist, die zur Herstellung eines elektrischen Kontaktes mit dem Körper des Patienten ohne zusätzliche Anwendung von separaten Kontaktmitteln geeignet ist.

2. Elektrode nach Anspruch 1, dadurch gekennzeichnet, daß der Kunststoff-Schaumstoffeinsatz (3; 9—12; 29) aus einem geschlossenporigen Schaumstoff aus leitfähigem Basismaterial besteht.

3. Elektrode nach Anspruch 1, dadurch gekennzeichnet, daß der Kunststoff-Schaumstoffeinsatz (3; 9—12; 29) aus einem offenporigen, nach dem Aufschäumen beleitfähigtem Schaumstoff besteht, dessen als Kontaktfläche vorgesehene Oberfläche eine porenverschließende Lackierung aufweist.

4. Elektrode nach Anspruch 1, dadurch gekennzeichnet, daß der Kunststoff-Schaumstoffeinsatz (3; 9—12; 29) einen spezifischen elektrischen Widerstand im Bereich kleiner als 2000 Ohm · cm und eine Stauchhärte im Bereich von 1 bis 20 kPa aufweist.

5. Elektrode nach Anspruch 2, dadurch gekennzeichnet, daß der Kunststoff zur Bildung eines geschlossenporigen Schaumstoffes ein Silikonkautschuk ist.

6. Elektrode nach Anspruch 3, dadurch gekennzeichnet, daß der Kunststoff zur Bildung eines offenporigen Schaumstoffes ein Polyurethan, wie Polyäther oder Polyester, ein Polyäthylen, ein Polyvinylchlorid oder ein Polyamid ist.

7. Elektrode nach einem der Ansprüche 1 bis 6, dadurch gekennzeichnet, daß der Kunststoff-Schaumstoffeinsatz (3, 29) an einer leitenden Trägerplatte, vorzugsweise aus Graphit oder Leitgummi, gehaltert ist.

8. Elektrode nach einem der Ansprüche 1 bis 6, dadurch gekennzeichnet, daß der Kunststoff-Schaumstoffeinsatz (3 bzw. 9 bis 12) an einer nichtlei tenden Elektrodenträger (2 bzw. 8), vorzugsweise aus Moosgummi, unter Zwischenschaltung einer elektrischen Leitungskontaktierung (4 bzw. 15 etc.) gehaltert ist.

9. Elektrode nach Anspruch 8, dadurch gekennzeichnet, daß die elektrische Kontaktierung (4 bzw. 15 etc.) eine Metallgaze ist, an der die elektrische Leitung (5 bzw. 16 bis 19) zur Abnahme oder Zuführung der elektrischen Signale angeschlossen ist.

10. Elektrode nach einem der Ansprüche 1 bis 9, dadurch gekennzeichnet, daß in Ausbildung der Elektrode als Mehrfachelektrode (Fig. 2) eine der gewünschten Zahl der Kontaktstellen entsprechende Anzahl von Kunststoff-Schaumstoffeinsätzen (9 bis 12) auf einem gemeinsamen Elektrodenträger (8) angeordnet sind.

11. Elektrode nach Anspruch 10, dadurch gekennzeichnet, daß der von Kunststoff-Schaumstoffeinsätzen (9 bis 12) freie Zwischenraum zwischen Elektrodenträger (8) und einem gemeinsamen Elektrodenpapier (13) durch nichtleitende Einsätze, vorzugsweise aus Moosgummi, ausgefüllt ist.

12. Elektrode nach Anspruch 8, dadurch gekennzeichnet, daß in Sandwichbauweise zwischen Moosgummi als Elektrodenträger und dem die Zwischenräume füllenden Moosgummi an den Verbindungsstellen zu den Kunststoff-Schaumstoffeinsätzen (9 bis 12) als Leitverbindung Metallgaze (15 etc.) liegt, an der jeweils elektrische Leitungen (16 bis 19) zur Abnahme bzw. Zuführung von elektrischen Signalen ankontaktiert sind.

13. Elektrode nach einem der Ansprüche 1 bis 9, dadurch gekennzeichnet, daß in Ausbildung der Elektrode als Saugelektrode mit einem Saugnapfgehäuse (21) mit Anschluß an einen Unterdruckerzeuger der Kunststoff-Schaumstoffeinsatz (29) im Saugnapfgehäuse (21) mit der applikationsseitigen porenverschlossenen Kontaktfläche (30) zur Herstellung des elektrischen Kontaktes bei Applikation angeordnet ist.

14. Elektrode nach Anspruch 13, dadurch gekennzeichnet, daß eine leitende Elektrodenträgerplatte (27), vorzugsweise aus Graphit oder Leitgummi, zur Halterung des Kunststoff-Schaumstoffeinsatzes (29) im Napfgehäuse (21) angeordnet ist.

15. Elektrode nach Anspruch 13, dadurch gekennzeichnet, daß wenigstens jene Teile zur Unterdruckerzeugung im Saugnapfgehäuse (21), die mit engen Saugdüsen (25) versehen sind, aus Kunststoff gefertigt sind.

16. Elektrode nach Anspruch 13, dadurch gekennzeichnet, daß bei Anwendung des Luftstrahlpumpenprinzips wenigstens das Strahlrohr (22) im Saugnapfgahäuse (21) als Kunststoffspritzteil ausgebildet ist.

**Claims**

1. An electrode for picking up electrical signals from or supplying electrical signals to the body of a patient, comprising at least one contact portion consisting of an electrically conductive synthetic resin foam insert (3; 9—12; 29), characterised in that on the application side, the synthetic resin foam insert (3; 9—12; 29) has a smooth, closed pore surface, serving as a contact surface (6; 14; 30), which is suitable for establishing electrical contact with the body of the patient without the additional use of separate contact means.

2. An electrode as claimed in Claim 1, characterised in that the synthetic resin foam insert (3; 9—12; 29) consists of a closed-pore foam made of a conductive basic material.

3. An electrode as claimed in Claim 1, characterised in that the synthetic resin foam insert (3; 9—12; 29) consists of an open-pore foam which is rendered conductive after foaming, the surface of said insert which serves as the contact surface being provided with a closed-pore lacquer coating.

4. An electrode as claimed in Claim 1, characterised in that the synthetic resin foam insert (3; 9—12; 29) has a specific electrical resistance in the range of less than 2000 Ohm · cm and a compression hardness in the range of from 1 to 20 kPa.

5. An electrode as claimed in Claim 2, characterised in that the synthetic resin used to form a closed-pore foam is a silicone rubber.

6. An electrode as claimed in Claim 3,

characterised in that the synthetic resin used to form a closed-pore foam is a polyurethane, such as polyether or polyester-urethane, a polyethylene, a polyvinyl chloride, or a polyamide.

7. An electrode as claimed in one of Claims 1 to 6, characterised in that the synthetic resin foam insert (3, 29) is supported on a conductive carrier plate, preferably made of graphite, or conductive rubber.

8. An electrode as claimed in one of Claims 1 to 6, characterised in that the synthetic resin foam insert (3 and 9 to 12) is supported on a non-conductive electrode carrier (2 and 8), preferably consisting of sponge rubber, with an interposed contact (4 and 15 etc.) for an electrical lead.

9. An electrode as claimed in Claim 8, characterised in that the electrical contact (4 and 15 etc.) consists of metal gauze to which the electrical lead (5 and 16 to 19) is connected for the pick-up or supply of the electrical signals.

10. An electrode as claimed in one of Claims 1 to 9, characterised in that, when the electrode is formed as a multiple electrode (Fig. 2), a number of synthetic resin foam inserts (9 to 12) corresponding to the desired number of contact points, are arranged on a common electrode carrier (8).

11. An electrode as claimed in Claim 10, characterised in that the interspace between the electrode carrier (8) and a common electrode paper (13) which is free from synthetic resin foam inserts (9 to 12) is filled with non-conductive inserts, preferably made of foam rubber.

12. An electrode as claimed in Claim 8, characterised in that, as a sandwich structure, metal gauze (15 etc.) serving as a lead contact, is arranged between the sponge rubber serving as electrode carrier and the sponge rubber which fills the interspaces at the connection points to the synthetic resin foam inserts (9 to 12), said metal gauze being connected to electrical lines (16 to 19) which serve to pick-up and supply electrical signals.

13. An electrode as claimed in one of Claims 1 to 9, characterised in that when the electrode is in the form of a suction electrode with a suction cup housing (21) connected to a reduced pressure generator, the synthetic resin foam insert (29) is arranged in the suction cup housing (21) with the closed-pore contact surface (30) on the application side in order to establish electrical contact on application.

14. An electrode as claimed in Claim 13, characterised in that a conductive electrode carrier plate (27), preferably made of graphite or conductive rubber, is provided to support the synthetic resin foam insert (29) in the cup housing (21).

15. An electrode as claimed in Claim 13, characterised in that at least those components for generating reduced pressure in the suction cup housing (21) which are provided with narrow suction nozzles (25) are made of synthetic resin.

16. An electrode as claimed in Claim 13, characterised in that, when the principle of the air jet pump is used, at least the jet tube (22) in the suction cup housing (21) is formed as a moulded synthetic resin component.

**Revendications**

1. Electrode destinée à prélever des signaux électriques du corps d'un patient ou à les y acheminer, comprenant au moins une pièce de contact en une garniture (3; 9 à 12; 29) en matière plastique mousse, conductrice de l'électricité, caractérisée en ce que la garniture (3; 9 à 12; 29) en matière plastique mousse présente du côté de l'application une surface lisse à pores fermés servant de surface (6; 14; 30) de contact, qui convient pour l'obtention d'un contact électrique avec le corps du patient, sans avoir à utiliser des moyens de contact distincts supplémentaires.

2. Electrode suivant la revendication 1, caractérisée en ce que la garniture (3; 9 à 12; 29) en matière plastique mousse est en une matière mousse, à pores fermés, en une substance de base conductrice.

3. Electrode suivant la revendication 1, caractérisée en ce que la garniture (3; 9 à 12; 29) en matière plastique mousse est en une matière mousse à pores ouverts rendue conductrice par moussage, dont la surface destinée à servir de surface de contact présente un laquage fermant les pores.

4. Electrode suivant la revendication 1, caractérisée en ce que la garniture (3; 9 à 12; 29) en matière plastique mousse a une résistance électrique spécifique inférieure à 2000 ohm·cm environ et une résistance à l'écrasement comprise entre 1 et 20 kPa environ.

5. Electrode suivant la revendication 2, caractérisée en ce que la matière plastique, qui forme une matière mousse à pores fermés, est un caoutchouc de silicone.

6. Electrode suivant la revendication 3, caractérisée en ce que la matière plastique qui forme une matière mousse à pores ouverts est un polyuréthane, comme un polyéther ou un polyester, un polyéthylène, un poly(chlorure de vinyle) ou un polyamide.

7. Electrode suivant l'une des revendications 1 à 6, caractérisée en ce que la garniture (3, 29) en matière plastique mousse est portée par une plaque support conductrice, de préférence en graphite ou en caoutchouc conducteur.

8. Electrode suivant l'une des revendications 1 à 6, caractérisée en ce que la garniture (3 ou 9 à 12) en matière plastique mousse est portée par un porte-électrodes (2 ou 8) non conducteur, de préférence en caoutchouc mousse, avec interposition d'un élément de contact (4 ou 15, etc) conducteur de l'électricité.

9. Electrode suivant la revendication 8, caractérisée en ce que l'élément (4 ou 15, etc) de contact électrique est une gaze métallique à laquelle est raccordé le conducteur (5 ou 16 à 19) électrique destiné à prélever les signaux électriques ou à les acheminer.

10. Electrode suivant l'une des revendications 1 à 9, caractérisée en ce que l'électrode étant agencée en électrodes multiples (figure 2), un nombre de garnitures (9 à 12) en matière plastique mousse correspondant au nombre souhaité d'endroits de contact est disposé sur un porte-électrodes (8) commun.

11. Electrode suivant la revendication 10, caractérisée en ce que l'espace intermédiaire exempt d'inserts (9 à 12) en matière plastique mousse, compris entre le porte-électrodes (8) et un papier (13) commun d'électrodes, est empli de charges non conductrices, de préférence en caoutchouc mousse.

12. Electrode suivant la revendication 8, caractérisée en ce qu'est interposée à la manière d'un stratifié entre le caoutchouc mousse servant de porte-électrodes et le caoutchouc mousse remplissant les espaces intermédiaires, aux endroits de liaison aux garnitures (9 à 12) en matière plastique mousse, de la gaze (15, etc.) métallique servant de liaison conductrice, sur laquelle viennent en contact les conducteurs (16 à 19) électriques destinés à prélever des signaux électriques et à les acheminer.

13. Electrode suivant l'une des revendications 1 à 9, caractérisée en ce que l'électrode étant une électrode à succion ayant un godet (21) d'aspiration raccordé à un générateur de dépression, la garniture (29) en matière plastique mousse est disposée lors de l'application dans le godet (21) d'aspiration pour obtenir le contact électrique en ayant les faces (30) de contact à pores fermés se trouvant du côté de l'application.

14. Electrode suivant la revendication 13, caractérisée en ce qu'une plaque (27) porte-électrodes conductrice, de préférence en graphite ou en caoutchouc conducteur, est prévue en vue de retenir la garniture (29) en matière plastique mousse dans le godet (21).

15. Electrode suivant la revendication 13, caractérisée en ce qu'au moins chaque pièce, qui est destinée à l'obtention de la dépression dans le godet (21) et qui est munie de buses (25) d'aspiration étroites, est en matière plastique.

16. Electrode suivant la revendication 13, caractérisée en ce que, lorsque l'on utilise le principle de la pompe à jet d'air, au moins le tube (22) pour le jet est agencé dans le godet (21) d'aspiration sous forme de pièce en matière plastique moulée par injection.

FIG 1

FIG 2

FIG 3

FIG 4